(19) Europäisches Patentamt
European Patent Office
Office européen des brevets

(11) **EP 4 509 053 A1**

(12) **EUROPEAN PATENT APPLICATION**

(43) Date of publication:
**19.02.2025 Bulletin 2025/08**

(51) International Patent Classification (IPC):
**A61B 5/38** (2021.01)

(21) Application number: **24194858.7**

(52) Cooperative Patent Classification (CPC):
**A61B 5/125; A61B 5/38**

(22) Date of filing: **16.08.2024**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **18.08.2023 EP 23192154**

(71) Applicant: **Interacoustics A/S**
**5500 Middelfart (DK)**

(72) Inventor: **UNDURRAGA LUCERO, Jamie Andres**
**DK-5500 Middelfart (DK)**

(74) Representative: **Demant**
**Demant A/S**
**Kongebakken 9**
**2765 Smørum (DK)**

(54) **INSTRUMENT FOR DETECTING AUDITORY EVOKED NEURAL RESPONSES**

(57) The present application relates to an instrument for detecting evoked responses. The instrument comprises a stimulus generator configured to generate at least one stimulus or a plurality of consecutive stimuli according to a test protocol, at least one output unit comprising a transducer, the output unit being configured to receive said at least one stimulus or plurality of consecutive stimuli from said stimulus generator and to provide said at least one stimulus or plurality of consecutive stimuli the test subject, at least one recording unit comprising one or more sensors for measuring one or more evoked responses of the test subject, in response to said provided at least one stimulus or plurality of consecutive stimuli, an analysis unit configured to receive and analyse said measured one or more evoked responses, where said analysis unit is configured to determine a probability, p, of whether each of said responses is driven by an underlying background noise during operation of said instrument, and where the analysis unit is configured to determine said probability, p, based on an F-value of the measured one or more evoked responses determined as a ratio between a variance of the average one or more evoked responses and a variance of a residual background noise. The application further relates to a method of detecting evoked responses.

FIG. 1

**Description**

SUMMARY

The present application relates to an instrument for detecting evoked responses.

The present application further relates to a method of detecting evoked responses.

The present application further relates to a detector unit.

The present application further relates to a data processing system comprising a processor and program code means for causing the processor to perform at least some of the steps of the method.

The present application further relates to a computer program comprising instructions which, when the program is executed by an instrument, cause the instrument to carry out the steps of the method.

An Instrument

Objective detection of auditory evoked neural activity plays a critical role in diagnosis and early intervention of hearing loss in newborns and infants as well as in neural assessment of auditory function during surgery (such as cochlear implantation or acoustic neuroma removal).

For newborns and infants, hearing screening may be performed by means of auditory evoked neural responses (i.e., electrophysiological responses) recorded by electroencephalography (EEG) using electrodes placed on the scalp of the babies. These responses include the auditory brainstem response (ABR), cortical auditory potentials (CAP), auditory steady-state responses (ASSR), otoacoustic emissions (OAE), among others. By presenting sounds (stimuli), such as clicks, narrow-band chirps, or speech signals, among others, the evoked response may be obtained by averaging many presentations of such stimuli, so the background noise is reduced, and the evoked response is then observed.

Detection of evoked neural responses has been, for several decades, achieved either via visual inspections by professional audiologists or clinicians, or by means of statistical methods (i.e., objective detection). The latter relies on a statistical parameter and its associated probability which indicates the likelihood that the measured parameter is driven by the underlying background noise (null hypothesis), rather than representing an actual evoked response (alternative hypothesis).

These objective methods include parametric (F-test, Hotelling-T2), non-parametric (Q-sample), and machine learning based approaches. Each of these methods has advantages and disadvantages. For example, the F-test is very efficient and intuitive as it measures the ratio between the energy of a signal with noise and the energy of the noise, which is easy to understand for anyone. However, the disadvantage of the F-test is that it may not account for individual types of underlying background noise in the EEG recording, which leads to less accurate and slower detections.

The Q-sample is a non-parametric test and relies either on bootstrapping methods or precalculated statistical tables (usually derived from simulations using artificial noise signals). Whilst bootstrapping can lead to very good estimates of the underlying background noise, it is computationally expensive and it can be conservative, i.e., prone to detect less true positive responses, when the number of iterations used to bootstrap the data is limited. As a result, the Q-sample with bootstrapping cannot be used in real-time measurements with standard clinical computers. This can be partially resolved by comparing the Q-sample statistics against preestablished tables obtained via simulations with different types of noise. However, this approach may not account for the individual properties of the background noise in a real recording.

Furthermore, the statistical quantity derived by the Q-sample as well as with the Hotelling-T2 method are abstract and difficult to understand for clinicians.

Finally, machine-learning methods depend on the training datasets and are prone to replicate the biases inherited from the training datasets.

Over the years, there has been an increasing interest in developing detectors (or methods of detecting) which are easy to interpret, reliable in terms of statistical detection, and applicable to real-time situations. The impact of such developments will be important to produce optimal measurements (fast, reliable, and with controlled false positive rates), which in turn directly impact accuracy, efficiency, and cost for clinical centres.

Currently, such a detector or method of detecting neural responses has not been reported, and objective detection of brain activity is achieved by the above-mentioned methods despite their shortcomings.

Accordingly, there is a need for an improved objective detection of auditory evoked neural responses, or any other type of response relying on averaging to reduce the underlying background noise to detect a signal.

In an aspect of the present application, an instrument (device/apparatus, or detector of an instrument) for detecting evoked responses is provided.

For example, the instrument (device/apparatus, or detector of an instrument) may be suitable for detecting auditory evoked neural responses.

The instrument may further be configured to estimate a hearing ability of a test subject (patient).

The instrument may comprise a stimulus generator.

The stimulus generator may be configured to generate at least one stimulus. The at least one stimulus may be generated according to a test protocol.

The stimulus generator may be configured to generate a plurality of consecutive stimuli according to a test protocol.

The instrument may comprise at least one output unit.

The at least one output unit may comprise a transducer.

The output unit may be configured to receive said at least one stimulus or plurality of consecutive stimuli from said stimulus generator.

The output unit may be configured to provide/present said at least one stimulus or plurality of consecutive stimuli into an ear of the test subject via said transducer.

The output unit may be configured to provide/present said at least one stimulus or plurality of consecutive stimuli as tactile or visual stimulus/stimuli to the test subject.

The output unit may be configured to present an acoustic stimulus.

The output unit may be configured to present an electrical stimulus (stimulation).

The output unit may be configured to present a tactile stimulus.

The output unit may be configured to present a visual stimulus.

For example, auditory neural responses may be evoked with cochlear implants (electrical) or bone conductor devices.

For example, the transducer may comprise a receiver (loudspeaker) for presenting the stimulus as an acoustic signal to the test subject.

The output unit may comprise an ear probe(s), a (stand-alone) loudspeaker, or a headset. A headset may refer to an in-the-ear, on-the-ear, or over-the-ear headset, earphone, etc., which is configured to introduce audio into the ears of the user.

The instrument may comprise at least one recording unit comprising one or more sensors for being arranged on the scalp of the test subject.

For example, said sensors may comprise electrodes or optodes or magnetometers or microphones.

Accordingly, the instrument may comprise at least one recording unit comprising one or more electrodes or optodes or magnetometers or microphones for being arranged on the scalp of the test subject.

Accordingly, said one or more electrodes or optodes or magnetometers or microphones may be configured to measure one or more evoked responses of the test subject.

The one or more evoked responses may be measured in response to the provided at least one stimulus or plurality of consecutive stimuli.

The evoked responses may be electrophysiological responses (i.e., auditory evoked neural responses), visual evoked potentials, acoustically evoked responses (such as OAEs), etc.. The one or more electrodes may be configured to measure one or more electrophysiological responses of the test subject.

The one or more electrodes may be configured to measure one or more electrophysiological responses of the test subject, in response to (as a result of) said provided at least one stimulus or plurality of consecutive stimuli.

In other words, the one or more sensors may be configured (or prompted, e.g., by a control unit) to measure said responses (immediately or with a time delay) following that a stimulus is provided/presented into the ear of the test subject.

For example, one or more sensors may refer to anything from 1 to several hundreds of sensors. For example, the one or more optodes may be used for Functional near-infrared spectroscopy (fNIRS).

For example, the one or more magnetometers may be used for Magnetoencephalography (MEG).

The at least one recording unit may be configured to receive (and record) the one or more evoked responses measured by the one or more electrodes.

For example, said responses may be measured/recorded by electroencephalography (EEG) using said one or more electrodes placed on the scalp of the test subject.

For example, the instrument comprising the one or more electrodes may be used in any EEG system/instrument and may be extended to any other domain in which a response is to be obtained by averaging measured responses (i.e., incoming data) to repeated presentations of stimuli (e.g., vestibular evoked responses, evoked otoacoustic emissions, magnetoencephalography, etc).

The instrument may comprise an analysis unit.

The analysis unit may be configured to receive said measured one or more evoked responses. The analysis unit may be configured to receive said measured one or more evoked responses from said recording unit.

The analysis unit may be configured to analyse said measured one or more evoked responses. The analysis unit may be configured to determine a probability, p, of whether each of said responses is driven by an underlying background noise during operation (i.e., online) of said instrument.

The analysis unit may be configured to determine said probability, p, based on an F-value of the measured one or more evoked responses determined as a ratio between a variance of the average one or more evoked responses and a variance of the residual background noise, each with the statistical degrees of freedom (DOF) $v$ and r, respectively.

In other words, the analysis unit may be configured to estimate a probability, p, (i.e., a likelihood) of whether said measured response is a result of an underlying background noise in the measured response, instead of being evoked by a stimulus presented in the ear of the test subject.

In other words, the analysis unit may be configured to determine said probability, p, from an F-value of the measured one or more evoked responses determined as a ratio between a variance of the average evoked response(s) and a variance of the residual background noise. More specifically, said ratio may be a ratio between a variance of the average one or more evoked responses (with statistical degrees of freedom (DOF) v) and a variance of a residual background noise (with statistical DOF r).

Thereby, an improved objective detection of evoked responses is provided, which is able to account for the background noise (obtained from the test subject) during a measurement, and which decreases the required detection/-measurement time.

The provided solution may be implemented in real-time and results in better estimates of the underlying background noise signal than existing technologies.

The provided solution may be applied to any time-domain based evoked response (brainstem and cortical responses) and may be used for diagnostics with higher precision and improved detection times compared with existing solutions.

Further, the provided solution may be applied to any frequency-domain based evoked response (brainstem and cortical responses, for example, Auditory Steady-State Responses (ASSRs), Frequency Following Response (FFR), Otoacoustics Emissions (OAE), and other responses) and may be used for diagnostics with higher precision and improved detection times compared with existing solutions.

In other words, the above-mentioned determination of probability, $p$, may be carried out during operation of the instrument.

The one or more sensors (electrodes or optodes or magnetometers or microphones) may be configured to measure one or more evoked responses comprising a ratio of stimulus evoked average response variance to the residual background noise variance.

In other words, an evoked response, measured following the presentation of a stimulus, may reflect an underlying background noise (signal) combined with a response evoked by the stimulus (in case this is sensed by the test subject). Accordingly, the measured evoked response may comprise partly a stimulus evoked response and partly the underlying background noise, where the term partly covers a percentage of 0 or higher.

For example, we may measure/record evoked responses (e.g., EEG data) in response to presented sounds. At each time segment a sound is presented, and trials of duration $T$ may be recorded using one or several electrodes.

A trial may refer to the presentation of one stimulus into an ear of the test subject and the subsequent measurement of evoked response.

Accordingly, the measured one or more evoked responses may have a duration T.

For example, a stimulus could be presented repeatedly 1000 times every 40 ms (e.g., 0 ms, 40 ms, 80 ms, ...). The duration of the evoked response could be set to 20 ms long, after each stimulus onset. In this case there will be 1000 individual trials, each of them lasting 20 ms. The individual trials from the continuous data stream may be indicated as $s_1$(t), $s_2$(t), ..., $s_N$(t) and may be obtained for the analysis, where $N$ is the total number of recorded trials. We are interested in obtaining the stimulus evoked response (i.e., the evoked neural response) $X(t)$ which, for the purpose of simplicity, may be assumed to be the same for each presented stimulus. By doing so, the problem can be represented by

$$\begin{matrix} \mathbf{S}\,(t) & \mathbf{X}\,(t) & \mathbf{BN}\,(t) \\ \begin{bmatrix} s_1\,(t) \\ s_2\,(t) \\ \vdots \\ s_N\,(t) \end{bmatrix} = \begin{bmatrix} X\,(t) \\ X\,(t) \\ \vdots \\ X\,(t) \end{bmatrix} + \begin{bmatrix} BN_1\,(t) \\ BN_2\,(t) \\ \vdots \\ BN_N\,(t) \end{bmatrix} \end{matrix} \qquad (1)$$

Here, $X(t)$ is the stimulus evoked response (signal) and $BN_i(t)$ is the underlying background noise (signal), both as a function of time t.

To reduce the underlying background noise, the average evoked response of all individual trials is estimated by

$$\overline{s\,(t)} = \mathbf{w}^T \mathbf{S}\,(t) = \mathbf{w}^T \mathbf{X}\,(t) + \mathbf{w}^T \mathbf{BN}\,(t) = X\,(t) + RN\,(t) \qquad (2)$$

where $RN(t)$ is the residual background noise in the averaged response (i.e., the residual noise), and **w** corresponds to a vector of estimated weights used to average the data.

Time domain

In the case of standard averaging, in the time domain, w is given by

$$\mathbf{w}_s^T = \left[ \frac{1}{N} \cdots \frac{1}{N} \right] = [w_1, w_2, ..., w_N] \tag{3}$$

whilst in the case of weighted average, **w** is given by

$$\mathbf{w}_w^T = \frac{\left[ \frac{1}{\sigma_{\mathrm{BN}_1}^2} \cdots \frac{1}{\sigma_{\mathrm{BN}_K}^2} \right]}{\left( \sum_{i=1}^{K} \frac{1}{\sigma_{\mathrm{BN}_i}^2} \right)} = [w_1, w_2, ..., w_N] \tag{4}$$

where $\sigma_{\mathrm{BN}_K}^2$ is the variance of the underlying background noise in the measurement (recording).

The variance of the underlying background noise ($\sigma_{\mathrm{BN}_K}^2$) can be estimated either 1) as the average variance of the individual variances of fixed time-points tracked across trials, or 2) as the variance of the points within each trial.

For the first approach, $\sigma_{\mathrm{BN}_K}^2$ is estimated from a subset of trials by

$$\hat{\sigma}_{\mathrm{BN}_k}^2 = \frac{1}{P} \sum_{n=1}^{P} \frac{1}{N_k - 1} \sum_{i=k_0}^{k_0+N_k} \left( s_i\left(n\Delta t\right) - \overline{s\left(n\Delta t\right)} \right)^2 \tag{5}$$

where $N_k$ is the number of trials used to compute $\sigma_{\mathrm{BN}_K}^2$. Here, each of the $N_k$ trials used to compute $\sigma_{\mathrm{BN}_K}^2$ is assumed to be recorded under the same quasi-static underlying background noise, i.e., each of the $N_k$ trials is weighted by

$$w_k = \frac{\frac{1}{\sigma_{\mathrm{BN}_K}^2}}{\left( \sum_{i=1}^{K} \frac{1}{\sigma_{\mathrm{BN}_i}^2} \right)}$$

.

For the second approach, $\sigma_{\mathrm{BN}_K}^2$ is computed for each trial by

$$\hat{s}_k\left(t\right) = s_k\left(t\right) - \delta\overline{s\left(t\right)} \tag{1}$$

$$\hat{\sigma}_{\mathrm{BN}_k}^2 = \frac{1}{P-1} \sum_{n=1}^{P} \left( \hat{s}_k\left(n\Delta t\right) - \overline{\hat{s}_k\left(n\Delta t\right)} \right)^2 \tag{2}$$

$$\tag{6}$$

where P is the number of points used to estimate the underlying background noise and which can include from 1 to all points within a trial. $\delta$ can be 0 if the neural activity is much smaller than the underlying background noise, or 1 if the neural response is expected to be large at the trial level. For this approach, each trial has its own $\sigma^2_{BN_K}$.

Once $\sigma^2_{BN_K}$, and consequently the weights ($\mathbf{w_s}$ or $\mathbf{w_w}$) have been obtained, the residual background noise in the average evoked response may be estimated by

$$\sigma^2_{RN} = \sum_{i=1}^{N} w_i^2 \sigma^2_{BN_i} \tag{7}$$

Now, the signal-to-noise ratio (SNR) may be estimated by

$$SNR = \frac{\sigma^2_{s}}{\sigma^2_{RN}} - 1 = F - 1 \tag{8}$$

where $\sigma^2_{s}$ is the variance of the average recording $\overline{s(t)}$ (i.e., of the average evoked response), and **F** is the ratio between the $\sigma^2_{s}$ and $\sigma^2_{RN}$.

The significance of the SNR is now entirely depending on the F-value which is determined by the statistical degrees of freedom (DOF) of $\sigma^2_{s}(v)$, the numerator, and the DOF of $\sigma^2_{RN}(r)$, the denominator.

Accordingly, the solution provides a novel estimation of these parameters. The provided solution estimates these DOF ($v$ and $r$) for each individual measurement, on-line whilst the data is recorded (i.e., during operation of the instrument).

The analysis unit may be configured to determine said probability based on at least a statistical degree of freedom (DOF) of said variance. The probability may be determined during operation of said instrument.

In other words, the present application provides that the statistical DOF $v$ is estimated online.

The analysis unit may be configured to determine said probability at least based on the statistical DOF of the variance of the average in one or more evoked responses and the statistical DOF of the variance of the residual background noise in the measured one or more evoked responses.

The analysis unit may be configured to determine an F-value of the measured one or more evoked responses as a ratio between said variance of the average one or more evoked responses and the variance of the residual background noise, each with the statistical DOF $v$ and r, respectively.

For example, without going into a formal mathematical demonstration, the following DOF is derived for the numerator of the F-ratio

$$v = \frac{2 \left[ \sum_{i=1}^{N_\omega} \hat{G}_{xx}(\omega_i) \right]^2}{\sum_{i=1}^{N_\omega} \hat{G}_{xx}(\omega_i)^2} \tag{9}$$

$$\hat{G}_{xx}(\omega) = \frac{2}{T} \sum_{i=1}^{N} w_i |X_i(\omega, T)|^2 \tag{10}$$

Here, $v$ is the DOF of $\sigma^2_{s}$, $N_\omega$ is the number of frequency bins of $\hat{G}_{xx}(\omega)$, and $\omega_i$ is the angular frequency of each frequency bin.

$\hat{G}_{xx}(\omega)$ is the power spectral density and it is obtained by equation 10, which corresponds to the weighted average of the squared magnitude of the Fourier transform of each trial

$$\hat{s}_i(t) \xrightarrow{F} X_i(\omega, T) \ with \ 0 \le t \le T \tag{11}$$

with $\hat{s}_i(t) = s_i(t) - \delta \overline{s(t)}$, where $\delta$ is 0 in the case that neural activity is much smaller than the underlying background noise, or 1 if the neural response is expected to be large at the trial level.

The weights $w_i$ are given by equation 3 or equation 4.

The DOF of the denominator is given by

$$r = v \frac{P}{Tf_s} \frac{\left[\sum_{i=1}^{N} w_i\right]^2}{\sum_{i=1}^{N} w_i^2} = \frac{2\left[\sum_{k=1}^{N_\omega} \hat{G}_{xx}(\omega_k)\right]^2}{\sum_{k=1}^{N_\omega} \hat{G}_{xx}(\omega_k)^2} \frac{P}{Tf_s} \frac{\left[\sum_{i=1}^{N} w_i\right]^2}{\sum_{i=1}^{N} w_i^2} \tag{12}$$

where $P$ is the number of tracked point used to estimate the background noise, $fs$ is the sampling rate of the recorded data, and $T$ is the duration of each trial.

Note that $\hat{G}_{xx}(\omega)$ can by estimated using a longer time window $T'$ and then scaled down for a smaller analysis window of length $T$

$$\hat{G}_{xx}(\omega) = \frac{2}{T'} \sum_{i=1}^{L} w_i' |X_i(\omega, T')|^2 \tag{1}$$

$$v = v' \frac{T}{T'} \tag{2}$$

$$\tag{13}$$

with $v'$ computed using Equation 9.

Once the DOF of the numerator and denominator have been obtained, the probability that the computed F-value is driven by the underlying background noise can be computed by

$$p = 1 - F_{CDF}(F, v, r) \tag{14}$$

where $F_{CDF}(F, v, r)$ is the cumulative distribution function of an F-distributed random variable with DOF $v$ and $r$.

Accordingly, the analysis unit may be configured to determine said probability, $p$, by:

$$p = 1 - F_{CDF}(F, v, r)$$

where $F_{CDF}(F, v, r)$ is the cumulative distribution function of an F-distributed random variable with v being the statistical DOF of the variance of the average (measured) one or more evoked responses and $r$ being the statistical DOF of the variance of the residual background noise.

The provided solution may measure the F-ratio (F-value; i.e., energy of the average measurement over energy of the estimated residual background noise). The ongoing activity may be weighted based on changes in the underlying background activity. These weights, derived via the consistent tracking of multiple points across and/or within the trials in the measurement, may be used to estimate the energy of the residual background noise in the measurement (the denominator on the F-ratio). The energy in the numerator may be estimated from the variance of the ongoing averaged evoked response. The provided solution uses a novel method to obtain the correct statistical degrees of freedom (DOF) which determines the sensitivity to find a statistically significant evoked response. Furthermore, the provided solution may be used in real time and its sensitivity may be adjusted automatically for each individual case, providing optimal individualised precision. Since the F-value is a parametric quantity, with the parameters being the DOF in the numerator (here referred as v), and the DOF in the denominator of the ratio (referred as r), its performance relies entirely on the accuracy of the estimate of these two parameters.

Frequency domain

In the case of standard averaging, in the frequency domain, w is given by

$$\mathbf{w}_s^T = \left[\frac{1}{N} \cdots \frac{1}{N}\right] = [w_1, w_2, ..., w_N] \qquad (15)$$

whilst in the case of weighted average, w is given by

$$\mathbf{w}_w^T = \frac{\left[\frac{1}{\sigma_{\mathrm{BN}_1}^2} \cdots \frac{1}{\sigma_{\mathrm{BN}_N}^2}\right]}{\left(\sum_{i=1}^N \frac{1}{\sigma_{\mathrm{BN}_i}^2}\right)} = [w_1, w_2, ..., w_N] \qquad (16)$$

where $\sigma_{\mathrm{BN}_N}^2$ is the variance of the underlying (residual) background noise in the measurement (recording).

An important property that will be used in the next equations is that the variance of a signal in the time domain can be estimated in the frequency domain by means of Parseval's theorem as the sum of the power spectral density of all positive frequencies. This is shown below

$$\hat{\sigma}_x^2 = \frac{1}{P-1} \sum_{n=1}^{P} \left(x\left(t_n\right) - \overline{x}\right)^2$$

$$\approx \frac{1}{P\left(P-1\right)} \sum_{\omega_j > 0} 2\left|X\left(\omega_j\right)\right|^2$$

$$x(t) \xrightarrow{\mathcal{F}} X\left(\omega, T\right) \, with \, 0 \leq t \leq T \qquad (17)$$

Where $T$ is the duration of a trial (or an epoch), $\mathcal{F}$ denotes the Fourier transform, $P$ is the number of samples within each trial or a subset of samples (if the desired time window is smaller than an epoch).

Using this property, the variance of the frequency-specific underlying background noise ( $\sigma_{\mathrm{BN}_N}^2$ ) can be estimated in the frequency domain as the sum of the power spectral density components of the **frequencies (e.g., angular frequencies) of interest** as

$$\hat{s}_n\left(t\right) = s_n\left(t\right) - \overline{\delta s\left(t\right)}$$

$$\hat{s}_n(t) \xrightarrow{\mathcal{F}} X_n\left(\omega, T\right) \, with \, 0 \leq t \leq T$$

$$\sigma_{\mathrm{BN}_n}^2 = \frac{1}{P\left(P-1\right)} \sum_{\omega_j} 2\left|X_n\left(\omega_j\right)\right|^2 \, for \, \omega_j > 0 \qquad (18)$$

Where $T$ is the duration of the trial, $\mathcal{F}$ denotes the Fourier transform, $P$ is the number of samples within each trial (an epoch) or a subset of samples (if the desired time window is smaller than an epoch), and $\omega_j \in [\omega_1, \omega_2, ... , \omega_L]$ is **the set of angular frequencies of interest.** $\delta$ can be 0 if the evoked activity is much smaller than the background noise, or 1 if the evoked response is expected to be large at the trial level.

If all frequencies are used to estimate the variance in Equation 18, we will obtain the same variance as in the time domain. However, if only a subset of frequency components are used, we will obtain the variance due only to those frequencies, i.e., a frequency-specific variance.

Once $\sigma_{\mathrm{BN}_N}^2$ , and consequently the weights ($\mathbf{w}_s$ or $\mathbf{w}_w$) have been obtained, the residual background noise in the average response can be estimated by

$$\sigma_{RN}^2 = \sum_{i=1}^{N} w_i^2 \sigma_{BN_i}^2 \tag{19}$$

Now, the signal-to-noise ratio (SNR) can be estimated by

$$SNR = \frac{\sigma_s^2}{\sigma_{RN}^2} - 1 = F - 1 \tag{20}$$

where $\sigma_s^2$ is the frequency-specific variance of the average recording $\overline{s(t)}$ (i.e., of the average evoked response) given by

$$\overline{s(t)} \xrightarrow{\mathcal{F}} \overline{X}(\omega, T') \text{ with } 0 \leq t \leq T' \leq T$$

$$\sigma_s^2 = \frac{1}{P'(P'-1)} \sum_{\omega_j} 2 \left| \overline{X}(\omega_j) \right|^2 \text{ for } \omega_j > 0 \tag{21}$$

Where $T$ is the duration of the analysis window (with $P'$ samples), and $F$ is the ratio between the $\sigma_s^2$ and $\sigma_{RN}^2$.

The significance of the SNR is now entirely depending on the $F$-value which is determined by the statistical DOF of $\sigma_s^2(v)$, the numerator, and the DOF of $\sigma_{RN}^2(r)$, the denominator.

The novelty of the presented solution relies on the estimation of these parameters. The analysis unit may estimate these DOF ($v$ and $r$) for each individual recording/measurement and from the specific frequencies of interest, on-line whilst the data is recorded.

The following DOF for the numerator of the F-value (i.e., an F-ratio) may be derived

$$v = \frac{2 \left[ \sum_{\omega_j} \hat{G}_{xx}(\omega_j) \right]^2}{\sum_{\omega_j} \hat{G}_{xx}(\omega_j)^2} \tag{22}$$

$$\hat{G}_{xx}(\omega) = \frac{2}{T} \sum_{i=1}^{N} w_i |X_i(\omega, T)|^2 \tag{23}$$

Here, $v$ is the DOF of $\sigma_s^2$ for the angular frequencies of interest $\omega_j$.

$\hat{G}_{xx}(\omega)$ is the power spectral density and it is obtained by equation 23, which corresponds to the weighted average of the magnitude of the Fourier transform of each trial

$$\hat{s}_i(t) \xrightarrow{\mathcal{F}} X_i(\omega, T') \text{ with } 0 \leq t \leq T' \tag{24}$$

with $\hat{s}_i(t) = s_i(t) - \delta\overline{s(t)}$. Where $\delta$ can be 0 if the neural activity is much smaller than the underlying background noise, or 1 if the neural response is expected to be large at the trial level.

The weights $w_i$ are given by equation 15 or equation 16.

The DOF of the denominator is given by

$$r = v \frac{\left[ \sum_{i=1}^{N} w_i \right]^2}{\sum_{i=1}^{N} w_i^2} = \frac{2 \left[ \sum_{\omega_j} \hat{G}_{xx}(\omega_j) \right]^2}{\sum_{\omega_j} \hat{G}_{xx}(\omega_j)^2} \frac{\left[ \sum_{i=1}^{N} w_i \right]^2}{\sum_{i=1}^{N} w_i^2} \tag{25}$$

Note that in many applications the duration of the analysis window used to estimate $\sigma_s^2$ ($P'$ samples) will be the same as that used to estimate $\sigma_{RN}^2$ ($P$ samples) in order to ensure the same frequency resolution for the estimation of the F-value.

Once the DOF of the numerator and denominator have been obtained, the probability that the computed F-value is driven by the underlying background noise can be computed by

$$p = 1 - F_{CDF}(F, v, r) \tag{26}$$

where $F_{CDF}(F, v, r)$ is the cumulative distribution function of an F-distributed random variable with DOF $v$ and $r$.

Accordingly, the analysis unit may be configured to determine said probability, p, by:

$$p = 1 - F_{CDF}(F, v, r)$$

where $F_{CDF}(F, v, r)$ is the cumulative distribution function of an F-distributed random variable with v being the statistical DOF of the variance of the average (measured) one or more evoked responses and r being the statistical DOF of the variance of the residual background noise.

There are many applications in which one or several frequencies need to be evaluated to assess if their power is significantly larger than that of the residual background noise, e.g., ASSRs. Steady-state visual evoked potentials, etcetera.

To deal with this problem, one could use the above given equations. However, if the number of frequencies to be tested is small (i.e., a small sparse number of frequencies of interest), the estimation of the residual background noise can be imprecise due to the limited number of data points (frequency samples) contributing to the estimation of the background noise. In order to solve this problem, the following adaptation can be used.

For example, consider that there are L sparsely distributed frequencies of interest ($\omega_1$, $\omega_2$, ..., $\omega_L$).

Next, consider that we want to assess whether the pooled power of these frequencies is significantly larger than the underlying background noise for the same frequencies or not.

Because of the low number of frequencies to be analyzed, relative to the full spectrum of the data, the estimation of the background noise variance (equation 18) can be imprecise. To avoid this problem, we can exploit the assumption that the power of the frequencies of interest should be similar to that of their neighboring frequencies. Under this assumption, we can obtain a good approximation of the power of the underlying background noise at each frequency of interest by averaging the power of frequencies in their neighborhood. This is:

$$\sigma_{BN_n}^2 = \frac{1}{P(P-1)} \sum_{\omega_l} 2\overline{|X_l(\omega_l)|^2} \text{ for } \omega_l > 0 \tag{1}$$

$$\overline{|X_l(\omega_l)|^2} = \frac{1}{N_{\omega_l}} \sum_{k=\omega_l-\Delta_{w_l}}^{\omega_l+\Delta_{w_l}} |X_n(\omega_l)|^2 \tag{2}$$

$$\tag{27}$$

Where $N_{\omega l}$ is the number of frequency bins around each frequency of interest $\omega_l$ used to estimate $\overline{|X_l(\omega_l)|^2}$.

Note that in equation 27 the number of samples and, therefore, the number of DOF will be higher as more samples contributed to this estimation.

The number of DOF given by equation 25 should now include all the frequencies used to estimate the variance of the underlying background noise in equation 27. This is, r may be determined by:

$$r = v_{BN} \frac{\left[\sum_{i=1}^{N} w_i\right]^2}{\sum_{i=1}^{N} w_i^2} = \frac{2\left[\sum_{\omega_j} \hat{G}_{xx}(\omega_j)\right]^2}{\sum_{\omega_j} \hat{G}_{xx}(\omega_j)^2} \frac{\left[\sum_{i=1}^{N} w_i\right]^2}{\sum_{i=1}^{N} w_i^2}$$

$$\omega_j \in [\omega_1 - \Delta\omega_1, \ldots, \omega_1 + \Delta\omega_1, \omega_2 - \Delta\omega_2, \ldots, \omega_2 + \Delta\omega_2, \ldots,$$

$$\omega_L - \Delta\omega_L, \ldots, \omega_L + \Delta\omega_L]$$

$$\tag{28}$$

Thereby, equation 26 can once again be used to estimate the probability that the computed F-value is driven by the residual background noise (and thereby by the underlying background noise).

In other words, in case the instrument is used in an ASSR measurement, equation 27 may be used to estimate $\sigma^2_{\mathrm{BN}_N}$. For example, if the response frequency is 40 Hz, $\sigma^2_{\mathrm{BN}_N}$ can be estimated as the average energy within the frequency interval 40 Hz $\pm$ 5 Hz.

The provided solution may further measure the F-ratio (energy of the average measurement over energy of the estimated residual background noise) in a frequency-specific manner. The ongoing activity may be weighted based on changes in the underlying background activity. These weights may be derived from the power in the frequency-domain and may be used to estimate the frequency-specific energy of the residual background noise in the measurement/recording (the denominator on the F-ratio). The energy in the numerator may be estimated from the power in the frequency domain of the ongoing averaged response. The provided solution may estimate the correct statistical degrees of freedom (DOF) based on the frequency components of interest which, in turn, determine the sensitivity to find a statistically significant evoked response. Furthermore, the provided solution may be used in real time and its sensitivity is adjusted automatically for each individual case, providing optimal individualised precision. Since the F-value is a parametric quantity, with the parameters being the DOF in the numerator (here referred as $v$), and the DOF in the denominator of the ratio (referred as $r$), its performance relies entirely on the accuracy of the estimate of these two parameters.

Accordingly, the analysis unit may be configured to estimate the variance of a frequency-specific underlying background noise ($\sigma^2_{\mathrm{BN}_N}$) as the sum of the power spectral density components of the frequencies (e.g., angular frequencies) of interest.

The analysis unit may be configured to estimate the residual background noise based on the variance of a frequency-specific underlying background noise ($\sigma^2_{\mathrm{BN}_N}$) and on the vectors of estimated weights ($\mathbf{w_s}$ or $\mathbf{w_w}$). In other words, the residual background noise variance ($\sigma^2_{\mathrm{RN}}$) in the average response can be estimated by equation 7 or equation 19.

The analysis unit may be configured to estimate the power of the residual background noise at each frequency of interest by averaging the power of the residual background noise of neighboring (adjacent) frequencies to said frequencies of interest.

The analysis unit may be configured to determine the numerator of the F-value based on DOF of $\sigma^2_s$ for the angular frequencies of interest $\omega_i$ (see equation 9 (time domain) and equation 22 (frequency domain)).

The analysis unit may be configured to determine the denominator of the F-value based on DOF of $\sigma^2_{\mathrm{RN}}$ for the angular frequencies of interest $\omega_i$ (see equation 12 (time domain) and equations 25 and 28 (frequency domain)).

The analysis unit may be configured to estimate the SNR, and accordingly the F-value, based on the ratio of $\sigma^2_s$ and $\sigma^2_{\mathrm{RN}}$ (see equation 20).

For example, $\sigma^2_s$ may be determined from equation 21(2).

For example, $\sigma^2_{\mathrm{RN}}$ may be determined from equation 19.

For example, the frequencies of interest may for a CAP measurement be 2 Hz to 30 Hz.

For example, the frequencies of interest may for an ASSR measurement be 40 Hz, 80 Hz, and further harmonics.

The analysis unit may be configured to adaptively determine said probability during operation of said instrument.

The probability may be determined either in the time domain and/or in the frequency domain. Thereby, a fast and continuous determination of the probability is provided to a user of the instrument while operating the instrument.

The at least one stimulus or plurality of consecutive stimuli may comprise acoustic stimuli.

The at least one stimulus or plurality of consecutive stimuli may comprise clicks.

The at least one stimulus or plurality of consecutive stimuli may comprise narrow-band chirps. The at least one stimulus or plurality of consecutive stimuli may comprise speech signals. The at least one stimulus or plurality of consecutive stimuli may comprise an electrical stimulation, or any repeated auditory stimulation.

For example, the at least one stimulus or plurality of consecutive stimuli may be based on the auditory change complex (ACC) stimuli.

The measured one or more evoked responses may comprise auditory brainstem responses (ABR).

The measured one or more evoked responses may comprise cortical auditory potentials (CAP). The measured one or more evoked responses may comprise auditory steady-state responses (ASSR).

For example, the measured one or more electrophysiological responses may comprise any response relying on averaging to reduce the background noise to detect a target signal, such as otoacoustics emissions, visual evoked responses, vestibular responses, among others.

The analysis unit may be configured to determine a hearing ability of a test subject in response to said probability being below a predetermined threshold.

The predetermined threshold may be, for example, the minimum stimulus amplitude to be able to hear a sound, the minimum difference to discriminate acoustics features between sounds, the minimum difference to discriminate speech in the presence of noise, the minimum difference to discriminate the spatial location of a sound, or the minimum difference to detect an odd sound from repeated ones.

The stimulus generator may be configured to amend said at least one stimulus or plurality of consecutive stimuli in response to said determined probability.

The stimulus generator may be configured to amend said test protocol in response to said determined probability.

The analysis unit may be configured to determine said stimulus or plurality of consecutive stimuli based on the analysis of previously measured evoked responses.

The analysis unit may comprise a neural network. The analysis unit may be configured to determine said probability, $p$, by use of said neural network.

Detector unit

In an aspect, a detector unit is furthermore provided by the present application.

The detector unit may be configured to determine a probability, $p$, of whether a measured evoked response is driven by an underlying background noise (signal).

For example, the above-described analysis unit may comprise said detector unit.

The detector unit may be configured to determine said probability, $p$, based on an F-value of the measured evoked response(s) determined as a ratio between a variance of the average evoked response(s) and a variance of a residual background noise, each with the statistical degrees of freedom (DOF) $\upsilon$ and $r$, respectively.

In other words, the detector unit may be configured to determine said probability, $p$, by:

$$p = 1 - F_{CDF}(F, v, r)$$

where $F_{CDF}(F, v, r)$ is the cumulative distribution function of an F-distributed random variable with $v$ being the statistical DOF of the variance of the average (measured) evoked response(s) and $r$ being the statistical DOF of the variance of the residual background noise.

Use

In an aspect, use of an instrument as described above, and in the claims, is moreover provided.

A method

In an aspect, a method of detecting evoked responses is furthermore provided by the present application.

For example, the method may be a method of detecting auditory evoked neural responses. The method may furthermore comprise estimating a hearing ability of a test subject.

The method may comprise generating at least one stimulus according to a test protocol.

The method may comprise generating a plurality of consecutive stimuli according to a test protocol.

The method may comprise providing said at least one stimulus to the test subject.

The method may comprise providing said at least one stimulus into an ear of the test subject via a transducer.

The method may comprise providing said plurality of consecutive stimuli to the test subject.

The method may comprise providing said plurality of consecutive stimuli into an ear of the test subject via a transducer.

The method may comprise arranging one or more electrodes on the scalp of a test subject.

The method may comprise arranging one or more optodes on the scalp of a test subject.

The method may comprise arranging one or more microphones in the ear of a test subject.

The method may comprise arranging one or more magnetometers on the scalp of a test subject.

The method may comprise measuring one or more evoked responses of the test subject.

The one or more evoked responses of the test subject may be measured/recorded, in response to (as a result of) said provided at least one stimulus or plurality of consecutive stimuli.

The method may comprise receiving said measured one or more evoked responses, by an analysis unit.

The method may comprise analysing said measured one or more evoked responses, by an analysis unit.

The analysis unit may determine a probability, p, of whether each of said responses is driven by a residual (underlying) background noise during operation of said instrument.

The analysis unit may determine said probability, p, based on an F-value of the measured one or more evoked responses determined as a ratio between a variance of the average one or more evoked responses and a variance of a residual background noise.

The analysis unit may determine said probability, *p*, based on an F-value of the measured one or more evoked responses determined as a ratio between a variance of the average one or more evoked responses and a variance of a residual background noise, each with the statistical degrees of freedom v and r, respectively.

For example, said analysis unit may determine said probability (in the time domain) by at least equations 7, 8, 9, 12 and 14.

For example, said analysis unit may determine said probability (in the frequency domain) by at least equations 19, 20, 21, 22, 25, 28, and 26.

It is intended that some or all of the structural features of the instrument described above, or in the claims can be combined with embodiments of the method, when appropriately substituted by a corresponding process and vice versa. Embodiments of the method have the same advantages as the corresponding instrument.

A computer readable medium or data carrier

In an aspect, a tangible computer-readable medium (a data carrier) storing a computer program comprising program code means (instructions) for causing a data processing system (a computer) to perform (carry out) at least some (such as a majority or all) of the (steps of the) method described above, and in the claims, when said computer program is executed on the data processing system is furthermore provided by the present application.

A computer program

A computer program (product) comprising instructions which, when the program is executed by a device (such as an instrument, a detector of an instrument, or a computer), cause the device to carry out (steps of) the method described above, and in the claims is furthermore provided by the present application.

A data processing system

In an aspect, a data processing system comprising a processor and program code means for causing the processor to perform at least some (such as a majority or all) of the steps of the method described above, and in the claims is furthermore provided by the present application.

A System

In a further aspect, a system comprising an instrument as described above, and in the claims, AND comprising an auxiliary device is moreover provided.

The system may be adapted to establish a communication link between the instrument and the auxiliary device to provide that information (e.g., control and/or status signals) can be exchanged or forwarded from one to the other.

The auxiliary device may comprise a remote control, a mobile device, a smartphone, or other portable electronic device, such as a tablet or the like, or may comprise a server device, etc.

The communication link may be a wired or wireless link, e.g., based on Bluetooth or some other standardized scheme.

An APP

In a further aspect, a non-transitory application, termed an APP, is furthermore provided by the present disclosure. The APP may comprise executable instructions configured to be executed, e.g., on the instrument or on the auxiliary device, e.g., to implement a control interface.

BRIEF DESCRIPTION OF DRAWINGS

The aspects of the disclosure may be best understood from the following detailed description taken in conjunction with the accompanying figures. The figures are schematic and simplified for clarity, and they just show details to improve the understanding of the claims, while other details are left out. Throughout, the same reference numerals are used for identical or corresponding parts. The individual features of each aspect may each be combined with any or all features of the other aspects. These and other aspects, features and/or technical effect will be apparent from and elucidated with reference to the illustrations described hereinafter in which:

FIG. 1 shows an example of an instrument for detecting auditory evoked neural responses.

FIG. 2 shows an example of a measurement and recording of electrophysiological responses by a recording unit comprising four electrodes.

FIG. 3 shows an example of a distribution of p-values in the presence of Gaussian noise as a function of trials.

FIG. 4 shows an example of a distribution of F-values in the presence of Gaussian noise as a function of trials.

FIG. 5 shows an example of a distribution of F-values in the presence of Gaussian noise and a simulated 'neural' signal as function of trials.

FIG. 6A and 6B show an example of another distribution of p-values in the presence of Gaussian noise as a function of trials.

FIG. 7A and 7B show an example of another distribution of p-values in the presence of Gaussian noise as a function of trials.

FIG. 8A and 8B show an example of detection rates in the presence of a simulated ASSR as function of the number of epochs.

Further scope of applicability of the present disclosure will become apparent from the detailed description given hereinafter. However, it should be understood that the detailed description and specific examples, while indicating preferred embodiments of the disclosure, are given by way of illustration only. Other embodiments may become apparent to those skilled in the art from the following detailed description.

DETAILED DESCRIPTION OF EMBODIMENTS

The detailed description set forth below in connection with the appended drawings is intended as a description of various configurations. The detailed description includes specific details for the purpose of providing a thorough understanding of various concepts. However, it will be apparent to those skilled in the art that these concepts may be practiced without these specific details. Several aspects of the instrument, system and method are described by various blocks, functional units, modules, components, steps, processes, algorithms, etc. (collectively referred to as "elements"). Depending upon particular application, design constraints or other reasons, these elements may be implemented using electronic hardware, computer program, or any combination thereof.

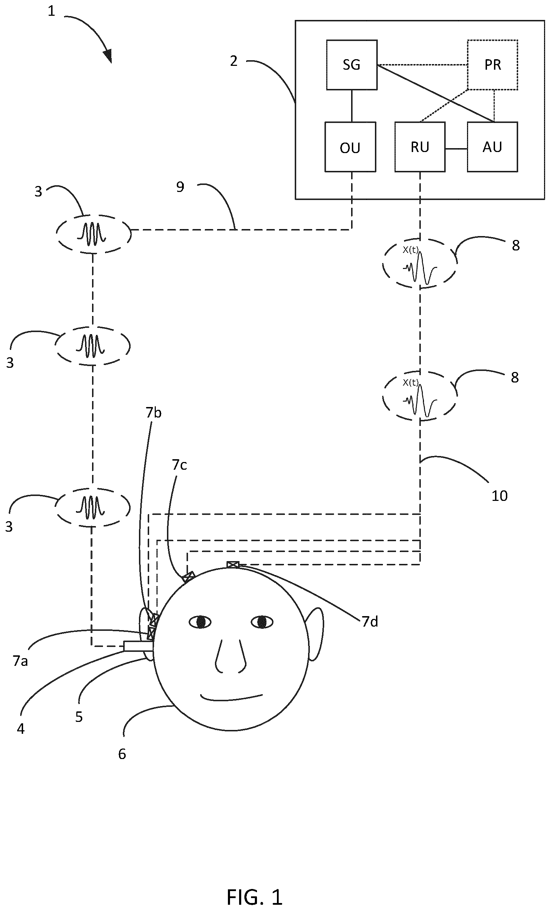

FIG. 1 shows an example of an instrument for detecting auditory evoked neural responses.

In FIG. 1, it is shown that the instrument 1 may comprise a stimulus generator SG, an output unit OU, a recording unit RU, and an analysis unit AU. The stimulus generator SG, output unit OU, recording unit RU, and analysis unit AU may be located in a base unit 2.

The stimulus generator SG may be configured to generate at least one stimulus 3 or a plurality of consecutive stimuli 3 according to a test protocol.

The output unit OU may be connected to the stimulus generator SG. The output unit OU may comprise a transducer. In FIG. 1, the transducer is arranged in an ear probe 4 and may comprise a receiver (loudspeaker) for providing the stimulus/stimuli 3 as an acoustic signal to an ear 5 of a test subject 6.

The output unit OU may be configured to receive said stimulus/stimuli 3 from said stimulus generator SG and provide said stimulus/stimuli 3 into an ear 4 of the test subject 6 via said transducer. It is shown that a plurality of consecutive stimuli 3 may be presented in the ear canal of the test subject 6 via the ear probe 4.

In FIG. 1, it is shown that the ear probe 4 is inserted into the right ear of the test subject 6, however it is foreseen that either the right ear or the left ear may be tested, or both ears may be tested simultaneously.

The recording unit RU may comprise one or more electrodes 7a,7b,7c,7d which are shown to be arranged/placed on the scalp of the test subject 6. The one or more electrodes 7a,7b,7c,7d may measure one or more electrophysiological responses 8 of the test subject 6 (for a time segment T), in response to one or a plurality of stimuli 3 are presented to the ear 5 of the test subject 6. The recording unit RU may record the electrophysiological responses 8.

The analysis unit AU may be configured to receive the measured (and recorded) one or more electrophysiological responses 8 from the recording unit RU (or alternatively, directly from the electrodes 7a,7b,7c,7d) (see the solid line between the RU and AU).

The analysis unit AU may be further configured to receive data/information (e.g., time, type, etc.) regarding the stimulus/stimuli 3 from the stimulus generator SG (see the solid line between SG and AU).

The analysis unit AU may be configured to analyse the one or more electrophysiological responses 8 measured by the electrodes 7a,7b,7c,7d. The analysis may be based on e.g., said data/information regarding the stimulus/stimuli 3 received from the stimulus generator SG. The analysis unit AU may be configured to determine a probability, p, of whether each of said responses is driven by an underlying background noise (or not). The determination may be further based on at least a variance of the measured one or more electrophysiological responses 8.

In FIG. 1, it is shown that the instrument 1 may additionally comprise a processor PR (e.g., located in the base unit 2). The processor PR may be configured to control the operation of the instrument 1, in other words, control that the test protocol is carried out and the measured data is analysed.

As indicated by the dotted lines, the processor PR may be connected to at least the stimulus generator SG, recording unit RU, and analysis unit AU.

As indicated by the dashed lines, the output unit OU may transmit the stimuli 3 via a wired or wireless connection 9 to the ear probe 4, and the recording unit RU may receive the measured electrophysiological responses 8 from the electrodes 7a,7b,7c,7d via a wired or wireless connection 10.

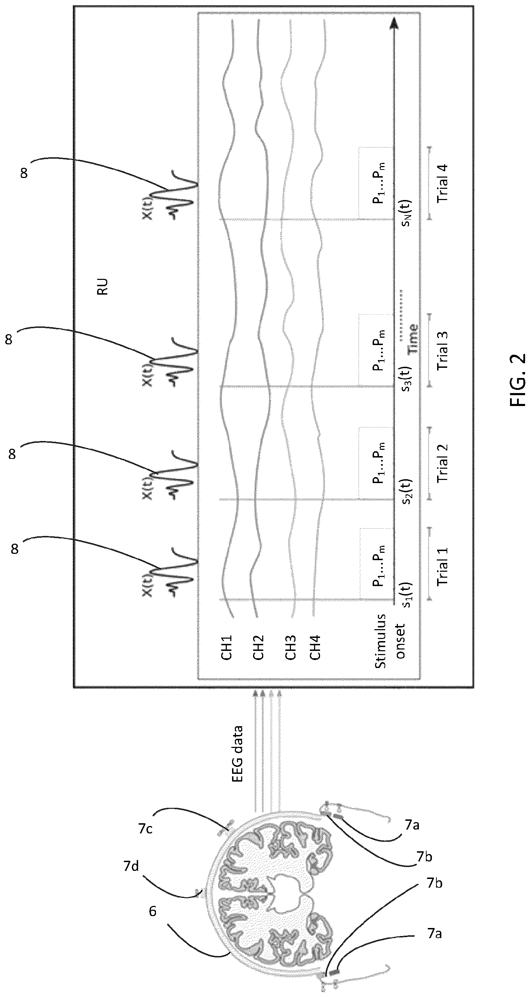

FIG. 2 shows an example of a measurement and recording of electrophysiological responses by a recording unit comprising four electrodes.

In FIG. 2, the electrophysiological responses may be EEG data. The EEG data may be measured by four electrodes 7a,7b,7c,7d arranged on the scalp of the test subject 6, substantially similar to the example shown in FIG. 1.

Accordingly, four channels may run from the electrodes 7a,7b,7c,7d to the recording unit RU for transmitting EEG data. The time series of the EEG data are illustrated in the graph in the recording unit RU.

The measured electrophysiological responses 8 (i.e., target neural responses) are shown by the (black) waveforms, $X(t)$, on the top of the graph.

The measured and recorded data, contaminated with underlying background noise, is shown for each channel by the individually coloured traces, i.e., the first (blue, CH1) and third (turquoise, CH3) curve from the top measured at the left ear, and the second (red, CH2) and fourth (orange, CH4) curve from the top measured at the right ear. The electrodes 7a,7b arranged at the left ear may represent channels CH1 and CH3, whereas the electrodes 7a,7b arranged at the right ear may represent channels CH2 and CH4. The electrode 7c arranged at the side and the electrode 7d arranged at the top of the scalp may represent a ground and a reference channel/electrode.

The onset of a new stimulus presentation is shown by the vertical lines in the graph, of which trials ($s_1(t)$, $s_2(t)$, ..., $s_N(t)$) of duration T are obtained.

The P's, $P_1$ ... $P_m$, indicate that a number, $m$, of points are tracked and used for determining the probability, $p$.

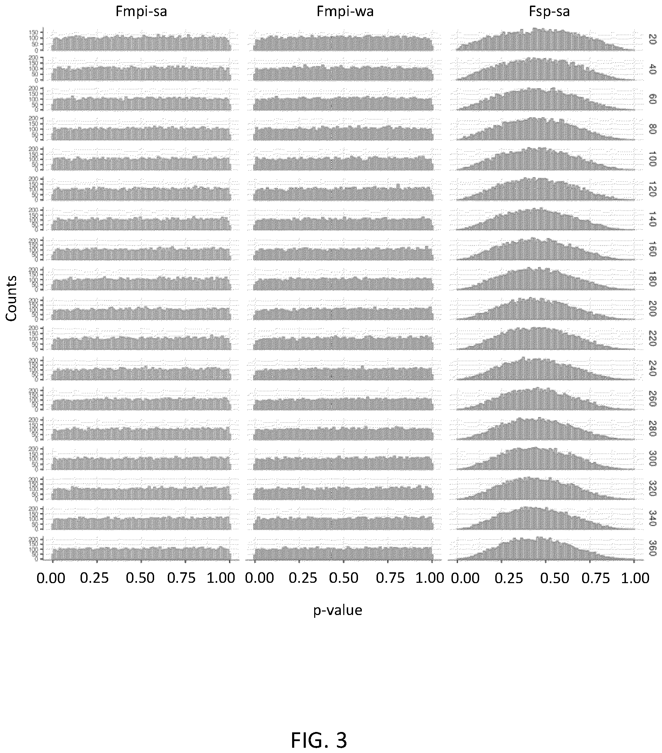

FIG. 3 shows the distribution of p-values in the presence of Gaussian noise as a function of trials.

Since the F-value is estimated using multiple (measurement) points, $m$, and the DOF (which characterize the underlying background noise for each recording, individually), the F-value determination is named Fmpi.

To illustrate the accuracy of the Fmpi detectors (or methods of detecting), a total of 10000 simulations were obtained each with 360 trials. The simulation was carried out using the typical setting of a cortical response recording. There was no neural response and the underlying background noise in the simulated recording was Gaussian. To compare the performance of the Fmpi detector, we processed the same datasets using the standard single-point F detector [1][2] with standard average (Fsp-sa). This detector uses a single point to estimate the residual noise of the F-ratio and assumes that the DOF is $r = N$ (the total number of trials) and $v = 5$ (based on empirical analysis of human EEG noise).

In FIG. 3, three different instruments (comprising three different detectors/methods for estimating the F-value) are used. The three different detectors (methods/algorithms) comprise an Fmpi using weighted average (Fmpi-wa), an Fmpi using standard average (Fmpi-sa), and an Fsp using standard average with $v = 5$ [1][2]. The trial length was 1 second, the sampling rate was 500 Hz, the analysis windows was 300 ms, and data was band-pass filtered between 2 and 30 Hz. All points within a trial were used by the Fmpi. Using the Fmpi detector, the p-values are uniformly distributed across all trials (which is the ultimate goal for a correct

estimation of the underlying noise distribution).

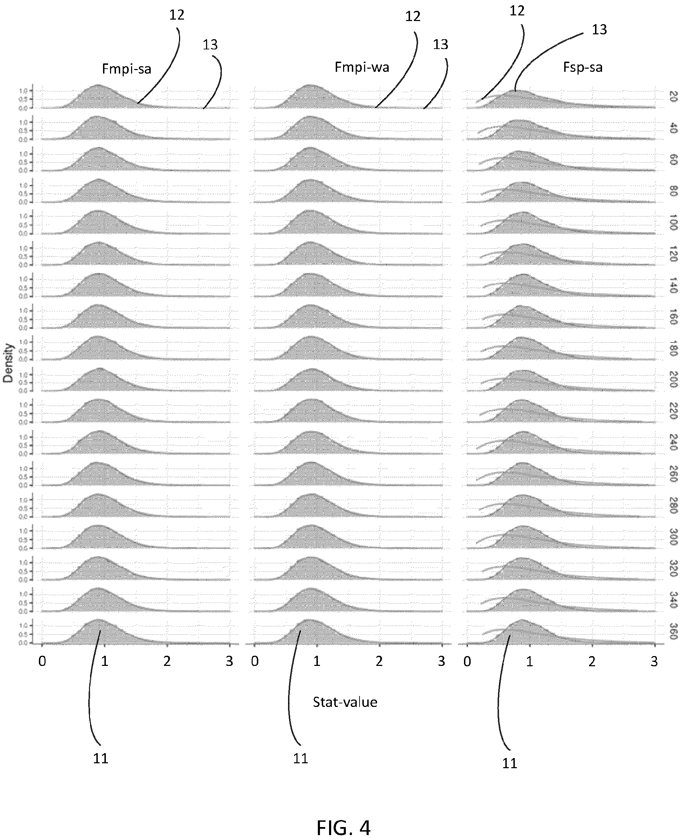

FIG. 4 shows an example of a distribution of F-values in the presence of Gaussian noise as a function of trials.

In FIG. 4, the histogram of the obtained F-values (stat-value, 11) are shown in grey. The probability density function estimated with three detectors (in blue, 12) is shown: the Fmpi using weighted average (Fmpi-wa), the Fmpi using standard average (Fmpi-sa), and the Fsp using standard average with $\upsilon = 5$ [1][2]. The empirical probability distribution is shown in red 13. The trial length was 1 second, the sampling rate was 500 Hz, the analysis windows was 300 ms, and data was band-pass filtered between 2 and 30 Hz. All points within a trial were used by the Fmpi detector.

The estimated F-distribution fits almost identically the empirical distribution of the data either with the Fmpi detector using standard (Fmpi-sa; Equation 3) or weighted (Fmpi-wa; Equation 4) average. On the other hand, a skewed distribution (FIG. 3; Fsp-sa) and poor fit (FIG. 4; Fsp-sa) is obtained with the classic F detector.

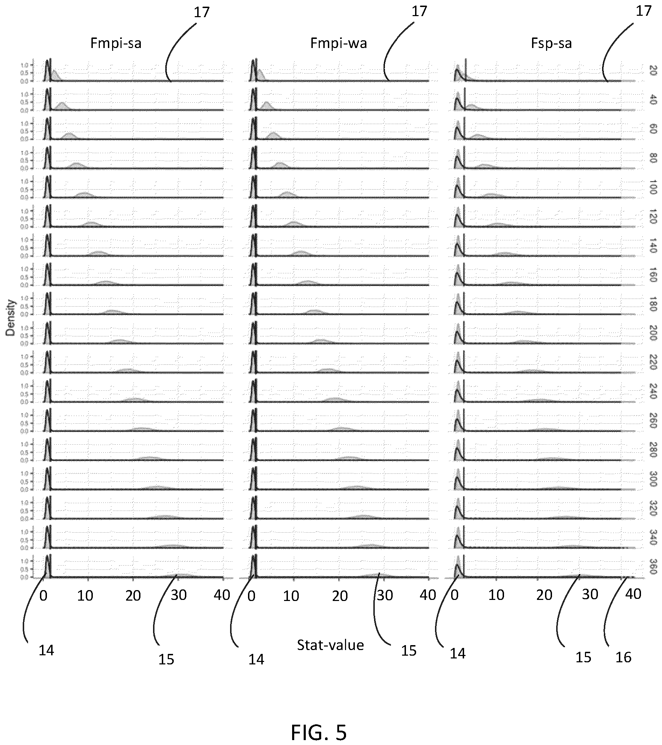

FIG. 5 shows an example of a distribution of F-values in the presence of Gaussian noise and a simulated 'evoked' signal as function of trials.

The simulation was carried out under the same conditions as for the previous example (FIGS. 3 and 4), but now including a target 'evoked' signal. The results show that the estimation of the underlying distribution of the noise was identical to that obtained without including a 'neural' response (compare solid with dashed black lines). The Fmpi detector correctly estimates the 5% chance point for a false positive detection (vertical line), resulting in earlier and more precise detections than the standard Fsp-sa detector, for which the '5%' chance point is overestimated (compare with empirical distribution) resulting in fewer detections at lower number of trials.

For comparison, the histogram of the F-values (stat-value) obtained without the target signal is shown in light-blue 14, whilst the histogram of F-values obtained in the presence of a target 'evoked' signal is shown in red 15, for all three detectors, i.e., the Fmpi using weighted average (Fmpi-wa), the Fmpi using standard average (Fmpi-sa), and the Fsp using standard average with [1] [2]. The underlying distribution of the noise obtained without the target neural signal is shown by the black dashed-line 16 whilst the distribution obtained in the presence of the target neural signal is shown by the black solid line 17. Vertical lines show the critical F-value to decide on the presence of a response. Values on the right side are considered detected responses. The trial length was 1 second, the sampling rate was 500 Hz, the analysis windows was 300 ms, and data was band-pass filtered between 2 and 30 Hz. The predetermined target SNR for the average of 360 trials was 6 dB. All points within a trial were used by the Fmpi detector.

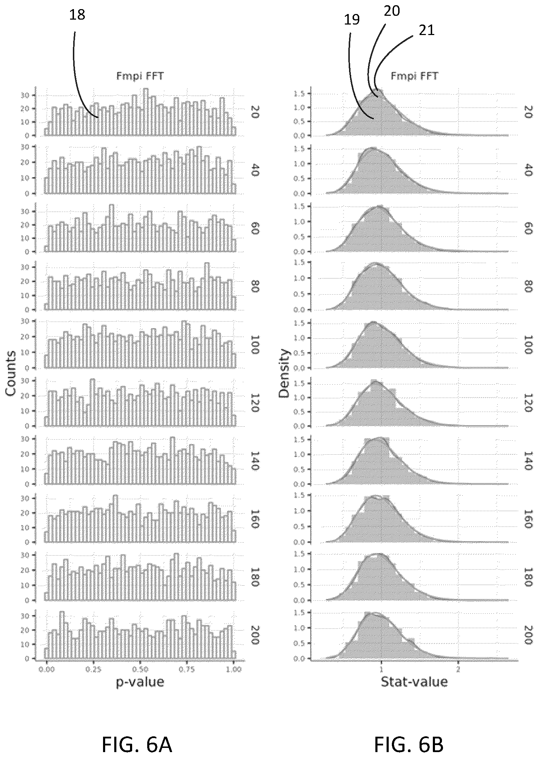

FIGs. 6A and 6B show an example of another distribution of p-values (probability values) in the presence of Gaussian noise as a function of trials.

To illustrate the accuracy determination of the F-value (and probability) in the frequency domain (e.g., by the detector unit), a typical recording session of 200 epochs was simulated. The probability of detecting a response in blocks of 20 epochs was estimated. Each block was run 1000 times, totalling 10000 random trials in the full simulation. The simulation was carried out using the typical setting of a cortical response recording. No neural response was included, so that it could be demonstrated how well the properties of the underlying background noise, which was Gaussian, can be predicted.

FIG. 6A shows the distribution of p-values of these simulations. The p-values are uniformly distributed across all trials (which is the ultimate goal for a correct estimation of the underlying distribution).

The distribution of F-values (stat-value) in the presence of Gaussian noise as a function of trials is shown in FIG. 6B. The histogram of the obtained F-values (stat-value) is shown in grey 19. The probability density function estimated with the modified detector is shown in blue (line 20). The empirical probability density is shown in red (line 21). The trial length was 1 second, the sampling rate was 500 Hz, the analysis windows was 500 ms, and data was band-pass filtered between 2 and 30 Hz. The estimated F-distribution fits almost identically the empirical distribution of the data.

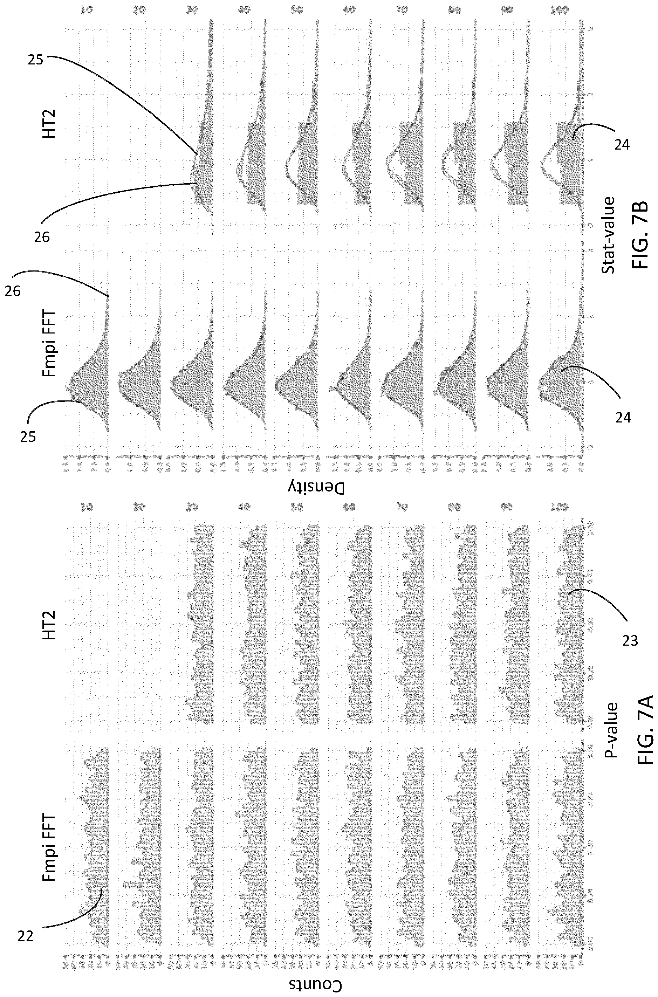

FIGs. 7A and 7B show an example of another distribution of p-values in the presence of Gaussian noise as a function of trials.

In FIG. 7A, the probability distribution (in the frequency-specific version; frequency domain) is tested by simulating an auditory steady-state response (ASSR). The simulation is carried out by convolving a template response at a rate of 41 Hz in the presence of Gaussian noise.

The results of this simulation is compared using the proposed solution (in the frequency domain) and the Hotelling-T-squared (HT2) tests, a typically used method for this type of analysis.

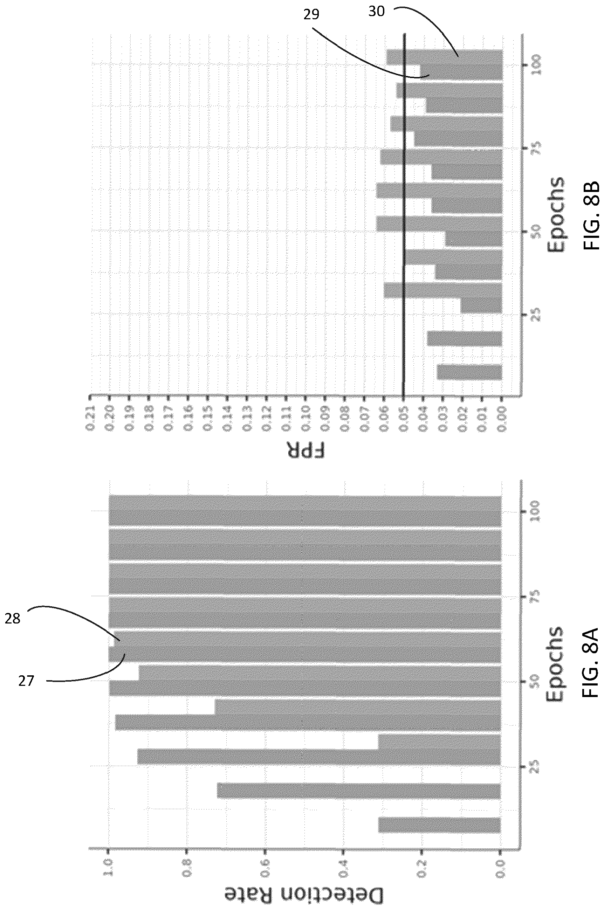

Both tested whether the compounded power of 12 frequencies (multiples 41 Hz) was significantly larger than that of the underlying background noise. Two simulations were run, first with no 'evoked response', i.e., only noise (FIGs. 7A and 7B), and next in presence of the simulated evoked response (FIG. 8A).

The results of the noise simulation show that both our solution and the HT2 test can predict well the underlying distributions. However, the HT2 method cannot produce reliable estimates when the number of frequencies of interests is larger than the number of trials included, which limits its usage, in particular, with a few number of trials.

In FIG. 7A, the distribution of p-values in the presence of Gaussian noise as a function of trials using the frequency-specific implementation of the proposed method (Fmpi-FFT) (red, 22) and the Hotelling-T-squared (HT2; blue, 23) test. FIG. 7B shows the distribution of F-values for the Fmpi-FFT and the HT2 tests in the presence of Gaussian noise as a function of trials. The histogram of the obtained F-values (stat-value) is shown in gray (24). The probability density function estimated with the Fmpi-FFT and the HT2 is shown in blue (line 25). The empirical probability distribution is shown in red (line 26). The HT2 method provides reliable estimations of the underlying distribution above 30 trails, but it fails to provide reliable estimates below 30 epochs, therefore those data points are not included. The trial length was 1 second, the sampling rate was 2000 Hz, the analysis windows was 1000 ms, and data is unfiltered.

FIGs. 8A and 8B shows an example of detection rates in the presence of a simulated ASSR as function of the number of epochs.

FIG. 8A shows detection rates for Fmpi-FFT 27 and HT2 28 in the presence of a simulated 41 Hz ASSR as a function of the number of epochs. The right graph (FIG. 8B) shows the false positive rates (FPR) for both the Fmpi-FFT 29 and the HT2 30 methods (same noise but without ASSR) as a function of the number of epochs. A total of 12 frequencies were included in the analysis (41 Hz and its harmonics). The trial length was 1 second, the sampling rate was 2000 Hz, the analysis windows was 1000 ms, and data was unfiltered. The horizontal line in the right panel denotes the target 5% false positive rate used in this simulation.

In FIG. 8A, it is seen that in the presence of a simulated neural signal, the Fmpi-FFT outperformed the HT2, showing more true detection (left graph) and less false positives (FIG. 8B) than the HT2 test.

It is intended that the structural features of the instrument and system described above, either in the detailed description and/or in the claims, may be combined with steps of the method, when appropriately substituted by a corresponding process.

As used, the singular forms "a", "an", and "the" are intended to include the plural forms as well (i.e., to have the meaning "at least one"), unless expressly stated otherwise. It will be further understood that the terms "includes", "comprises", "including", and/or "comprising", when used in this specification, specify the presence of stated features, integers, steps, operations, elements, and/or components, but do not preclude the presence or addition of one or more other features, integers, steps, operations, elements, components, and/or groups thereof. It will also be understood that when an element is referred to as being "connected" or "coupled" to another element, it can be directly connected or coupled to the other element, but an intervening element may also be present, unless expressly stated otherwise. Furthermore, "connected" or "coupled" as used herein may include wirelessly connected or coupled. As used herein, the term "and/or" includes any and all combinations of one or more of the associated listed items. The steps of any disclosed method are not limited to the exact order stated herein, unless expressly stated otherwise.

The claims are not intended to be limited to the aspects shown herein but are to be accorded the full scope consistent with the language of the claims, wherein reference to an element in the singular is not intended to mean "one and only one" unless specifically so stated, but rather "one or more". Unless specifically stated otherwise, the term "some" refers to one or more.

<u>REFERENCES</u>

[1] Don, M. and Elberling, C. (1994) 'Evaluating residual background noise in human auditory brain-stem responses.', The Journal of the Acoustical Society of America, 96(5 Pt 1), pp. 2746-57.

[2] Elberling, C. and Don, M. (1984) 'Quality estimation of averaged auditory brainstem responses.', Scandinavian audiology, 13(3), pp. 187-97.

**Claims**

**1.** Instrument for detecting evoked responses, where the instrument comprises

- a stimulus generator configured to generate at least one stimulus or a plurality of consecutive stimuli according to a test protocol,
- at least one output unit comprising a transducer, the output unit being configured to receive said at least one stimulus or plurality of consecutive stimuli from said stimulus generator and to provide said at least one stimulus or plurality of consecutive stimuli the test subject,
- at least one recording unit comprising one or more sensors for measuring one or more evoked responses of the test subject, in response to said provided at least one stimulus or plurality of consecutive stimuli,
- an analysis unit configured to receive and analyse said measured one or more evoked responses, where said analysis unit is configured to determine a probability, p, of whether each of said responses is driven by an underlying background noise during operation of said instrument, and where the analysis unit is configured to determine said probability, p, based on an F-value of the measured one or more evoked responses determined as a ratio between a variance of the average one or more evoked responses and a variance of a residual background noise.

2. Instrument according to claim 1, wherein said one or more sensors is configured to measure one or more evoked responses comprising a ratio of stimulus evoked average response variance to the residual background noise variance.

3. Instrument according to any one of the preceding claims, wherein said analysis unit is configured to determine said probability, *p*, by:

$$p = 1 - F_{CDF}(F, v, r)$$

where $F_{CDF}(F, v, r)$ is the cumulative distribution function of an F-distributed random variable with v being the statistical DOF of the variance of the average one or more evoked responses and r being the statistical DOF of the variance of the residual background noise.

4. Instrument according to any one of the preceding claims, wherein said analysis unit is configured to determine the numerator of the F-value based on DOF of $\sigma_s^2$ for the frequencies of interest $\omega_i$, and to determine the denominator of the F-value based on DOF of $\sigma_{RN}^2$ for the frequencies of interest $\omega_i$.

5. Instrument according to any one of the preceding claims, wherein the analysis unit is configured to estimate a power of the residual background noise at each frequency of interest by averaging the power of the residual background noise at neighboring frequencies to said frequencies of interest.

6. Instrument according to any one of the preceding claims, wherein said analysis unit is configured to adaptively determine said probability during operation of said instrument.

7. Instrument according to any one of the preceding claims, wherein said at least one stimulus or plurality of consecutive stimuli comprises acoustic stimuli such as clicks, narrow-band chirps, auditory change complex (ACC) type stimuli, and/or speech signals, and/or comprises electrical stimulations.

8. Instrument according to any one of the preceding claims, wherein said measured one or more evoked responses comprises auditory brainstem responses (ABR), cortical auditory potentials (CAP), or auditory steady-state responses (ASSR), or otoacoustic emissions (OAE), or any response relying on averaging to reduce the underlying background noise to detect a target signal.

9. Instrument according to any one of the preceding claims, wherein said analysis unit is configured to determine a hearing ability of a test subject in response to said probability being below a predetermined threshold.

10. Instrument according to any one of the preceding claims, wherein said stimulus generator is configured to amend said at least one stimulus or plurality of consecutive stimuli in response to said determined probability.

11. Method of detecting evoked responses, where the method comprises:

    - generating at least one stimulus or a plurality of consecutive stimuli according to a test protocol,
    - providing said at least one stimulus or plurality of consecutive stimuli to the test subject,
    - arranging one or more sensors on a test subject,
    - measuring one or more evoked responses of the test subject, in response to said provided at least one stimulus or plurality of consecutive stimuli,
    - receiving and analysing said measured one or more evoked responses, by an analysis unit,
    - determining a probability, *p*, of whether each of said responses is driven by an underlying background noise during operation of said instrument by determining said probability, *p*, based on an F-value of the measured one or more evoked responses determined as a ratio between a variance of the average one or more evoked responses and a variance of a residual background noise, each with the statistical degrees of freedom *v* and r, respectively.

12. A data processing system comprising a processor and program code means for causing the processor to perform at least some of the steps of the method of claim 11.

**13.** A computer program comprising instructions which, when the program is executed by an instrument according to any one of claim 1-10, cause the instrument to carry out the steps of the method according to claim 11.

**FIG. 1**

FIG. 2

FIG. 3

FIG. 4

FIG. 5

FIG. 6A

FIG. 6B

FIG. 7B

FIG. 7A

FIG. 8B

FIG. 8A

Europäisches
Patentamt

European
Patent Office

Office européen
des brevets

# EUROPEAN SEARCH REPORT

Application Number

EP 24 19 4858

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2003/144601 A1 (PRICHEP LESLIE S [US]) 31 July 2003 (2003-07-31) * paragraph [0034] * * figure 1 * | 1-13 | INV. A61B5/38 |
| A | M.S. JOHN ET AL: "MASTER: a Windows program for recording multiple auditory steady-state responses", COMPUTER METHODS AND PROGRAMS IN BIOMEDICINE, vol. 61, no. 2, 1 February 2000 (2000-02-01), pages 125-150, XP055215486, ISSN: 0169-2607, DOI: 10.1016/S0169-2607(99)00035-8 * the whole document * | 1-13 | |

TECHNICAL FIELDS
SEARCHED (IPC)

A61B

The present search report has been drawn up for all claims

| Place of search | Date of completion of the search | Examiner |
|---|---|---|
| The Hague | 25 September 2024 | Van Dop, Erik |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

.......................................................................

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 24 19 4858

This annex lists the patent family members relating to the patent documents cited in the above-mentioned European search report.
The members are as contained in the European Patent Office EDP file on
The European Patent Office is in no way liable for these particulars which are merely given for the purpose of information.

25-09-2024

| Patent document cited in search report | Publication date | Patent family member(s) | | Publication date |
|---|---|---|---|---|
| US 2003144601 A1 | 31-07-2003 | US | 2003144601 A1 | 31-07-2003 |
| | | WO | 2004078024 A2 | 16-09-2004 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **DON, M.** ; **ELBERLING, C.** Evaluating residual background noise in human auditory brain-stem responses. *The Journal of the Acoustical Society of America*, 1994, vol. 96 (5), 2746-57

- **ELBERLING, C.** ; **DON, M.** Quality estimation of averaged auditory brainstem responses. *Scandinavian audiology*, 1984, vol. 13 (3), 187-97